(19) Europäisches Patentamt
European Patent Office
Office européen des brevets

(11) Numéro de publication: **0 524 109 B1**

(12) **FASCICULE DE BREVET EUROPEEN**

(45) Date de publication de fascicule du brevet: **07.06.95**

(51) Int. Cl.[6]: **A61K 7/48**

(21) Numéro de dépôt: **92402079.5**

(22) Date de dépôt: **17.07.92**

(54) **Composition dépigmentante contenant des dérivés d'arbutoside.**

(30) Priorité: **19.07.91 FR 9109188**

(43) Date de publication de la demande:
**20.01.93 Bulletin 93/03**

(45) Mention de la délivrance du brevet:
**07.06.95 Bulletin 95/23**

(84) Etats contractants désignés:
**AT BE CH DE DK ES FR GB GR IT LI NL PT SE**

(56) Documents cités:
**FR-A- 2 577 805**

**PATENT ABSTRACTS OF JAPAN vol. 12, no. 82 (C-481)(2929)**

**PATENT ABSTRACTS OF JAPAN vol. 12, no. 209 (C-504)(3056)**

(73) Titulaire: **L'OREAL**
**14, Rue Royale**
**F-75008 Paris (FR)**

(72) Inventeur: **Pilleux, Eric**
**45, rue Boissonade**
**F-75014 Paris (FR)**
Inventeur: **Li, Ming**
**73, avenue du Général Leclerc**
**F-91190 Gif Sur Yvette (FR)**
Inventeur: **Cosson, Jean-Pierre**
**4, rue Lacave,**
**La Plagne**
**B.P. 643,**
**Nouméa,**
**Nouvelle-Caledonie (FR)**
Inventeur: **Guenard, Daniel**
**19, rue d'Arcueil**
**F-92120 Montrouge (FR)**
Inventeur: **Sevenet, Thierry**
**63, rue Letort**
**F-75018 Paris (FR)**
Inventeur: **Potier, Pierre**
**14, avenue de Breteuil**
**F-75007 Paris (FR)**

(74) Mandataire: **Stalla-Bourdillon, Bernard et al**
**Cabinet Nony**
**29, rue Cambacérès**
**F-75008 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 524 109 B1

Rank Xerox (UK) Business Services
(3.10/3.09/3.3.3)

## Description

La présente invention a pour objet une composition cosmétique ou dermatologique à action dépigmentante contenant en tant que principe actif au moins un dérivé d'arbutoside ou arbutine. La composition selon l'invention est tout particulièrement destinée à blanchir la peau ou à traiter les taches pigmentaires.

On rappellera que le mécanisme de formation de la pigmentation de la peau, c'est-à-dire de la formation des mélanines est particulièrement complexe et fait intervenir schématiquement les principales étapes suivantes :
Tyrosine —> Dopa —> Dopaquinone -----> Dopachrome -----> Mélanines l'enzyme intervenant dans cette suite de réactions étant essentiellement la tyrosinase.

Les substances les plus utilisées à l'heure actuelle en tant que dépigmentants sont plus particulièrement l'hydroquinone et ses dérivés, en particulier ses éthers tels que le monométhyléther d'hydroquinone.

Ces composés, s'ils ont une efficacité certaine, ne sont malheureusement pas exempts d'effets secondaires, ce qui peut rendre leur emploi délicat, voire dangereux.

Ainsi, l'hydroquinone, dont l'emploi est d'ailleurs limité à une concentration de 2 % , est un composé particulièrement irritant et cytotoxique pour le mélanocyte dont le remplacement, total ou partiel, a été envisagé par de nombreux auteurs.

Il a été ainsi proposé l'emploi de diverses substances naturelles dont l'arbutoside et le méthylarbutoside. Ces compositions à base d'arbutoside font l'objet du brevet français n° 85 04288 (2.577.805) et des demandes de brevet japonais n° 86/152,196 et n° 88/011,585.

Il est bien établi qu'une substance exerce une action dépigmentante si elle agit directement sur la vitalité des mélanocytes épidermiques où se déroule normalement la mélanogénèse, et/ou si elle interfère avec une des étapes de la biosynthèse des mélanines, soit en inhibant un des enzymes impliqués, soit en s'intercalant comme analogue structural dans la voie de synthèse qui peut ainsi être bloquée, d'où l'effet dépigmentant.

L'utilisation de substances dépigmentantes par voie topique présentant une bonne efficacité et étant inoffensive est tout particulièrement recherchée en vue de traiter les hyperpigmentations régionales par hyperactivité mélanocytaire telles que les mélasmas idiopathiques, survenant lors de la grossesse (masque de grossesse ou chloasma) ou secondaires à la contraception oestro-progestative, les hyperpigmentations localisées par hyperactivité et prolifération mélanocytaire bénigne telles que les taches pigmentaires séniles dites lintigo actiniques, les hyperpigmentations accidentelles telles que la photosensibilisation et la cicatrisation post-lésionnelle, ainsi que certaines leucodermies telles que le vitiligo ou, à défaut de pouvoir repigmenter la peau lésée, on achève de dépigmenter les zones de peau normale résiduelles pour donner à l'ensemble de la peau une teinte blanche homogène.

Après de nombreuses études sur diverses substances naturelles, on a constaté de façon tout à fait surprenante que certains dérivés de l'arbutoside avaient une action dépigmentante particulièrement prononcée bien supérieure à celle de l'arbutoside et d'autres substances dépigmentantes connues telles que l'hydroquinone ou l'acide 4-hydroxy-cinnamique.

Ces propriétés dépigmentantes ont pu être mises en évidence par le test d'inhibition "in vitro" de l'activité de la tyrosinase.

Les dérivés d'arbutoside selon l'invention se sont par ailleurs avérés présenter une très bonne tolérance cutanée, en particulier une absence d'effet irritant.

La présente invention a donc pour objet une composition cosmétique ou dermatologique à action dépigmentante contenant en tant que principe actif au moins un dérivé d'arbutoside correspondant à la formule générale suivante :

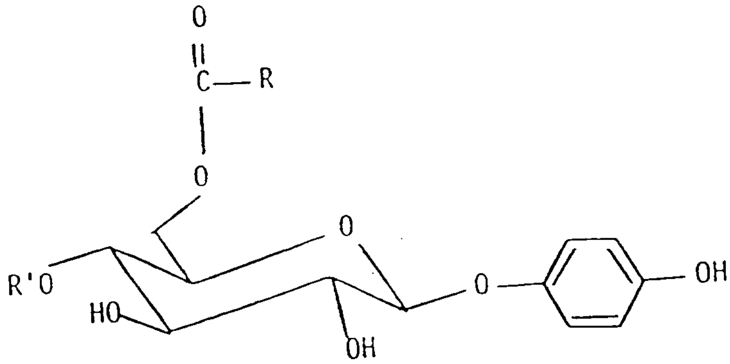

(I)

dans laquelle :

R représente un reste insaturé hydroxylé aliphatique ou aromatique ayant de 4 à 16 atomes de carbone, et

R' représente un atome d'hydrogène ou le radical

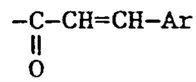

Ar représentant un radical phényle éventuellement substitué par un ou plusieurs groupes hydroxyles.

Selon une forme de réalisation préférée de l'invention, le reste insaturé hydroxylé aliphatique ou aromatique ayant de 4 à 16 atomes de carbone est choisi parmi les radicaux :

(i) 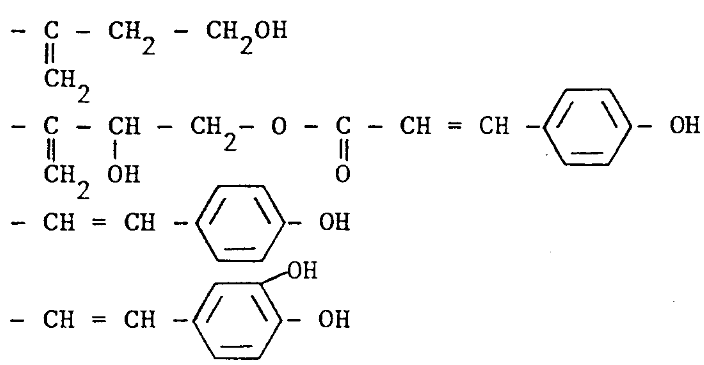

(ii)

(iii)

(iv)

Parmi les dérivés d'arbutoside des compositions selon l'invention, ceux particulièrement préférés sont les suivants :

Composés n°

(1) 6-p-coumaroylarbutoside,

(2) 6-caffeoylarbutoside,

(3) E,4-(p-hydroxy-cinnamoyl)-6-(4'''-hydroxy-2'''méthylène butanoyl)-arbutoside,

(4) E,4''-(p-hydroxy-cinnamoyloxy)-6-(3'',4''-dihydroxy-2''méthylène butanoyl)-arbutoside.

La plupart des composés de formule générale (I) des compositions selon l'invention ont été décrits dans la littérature (MANJU et coll. Phytochemistry, 1977, vol.16, p.793-794). Ils peuvent être obtenus soit par extraction à partir de plantes, ou encore par synthèse chimique par condensation sur l'arbutoside de l'acide insaturé hydroxylé dont le groupe hydroxy a été protégé par une fonction acétyle.

Ainsi la 6-p-coumaroylarbutoside est obtenue par condensation de l'arbutoside avec l'acide p-acétoxy cinnamique (E. HASLAM, Phenolic constituents of Vaccinium vitis-idaea, J.Chem.Soc, Suppl.I, 1964, 5649 ; M. VARMA et al, The synthesis of 2- and 6-0-p-coumaroyl and 6-0-p-hydroxybenzoyl arbutin derivatives, Monatsch. Chem., 1980, 111, 469 et T. IWAGAWA et al, Phenolic constituents of Viburnum carlesii, Kagoshima Daigaku Rigakubu Kiyo, Sugaku, Butsurigaku, Kagaku, 1988, 21, 89).

De même, la 6-caffeoylarbutoside est obtenue par condensation de l'arbutoside avec l'acide 3,4-diacétoxy cinnamique (K. MACHIDA, et al, Phenolic glycosides from Viburnum dilatatum, Phytochem., 1991,

30(6), 2013 et T. IWAGAWA et al, Phenolic constituents of Viburnum carlesii, Kagoshima Daigaku Rigakubu Kiyo, Sugaku, Butsurigaku, Kagaku, 1988, 21, 89).

En ce qui concerne les composés nouveaux (3) et (4) ci-dessus, ceux-ci ont été obtenus à partir d'une plante riche en ces composés appelée Garnieria Spathulaefolia.

On donnera ci-après dans la partie expérimentale, les conditions d'extraction de ces deux composés.

Les compositions selon l'invention contiennent en général une concentration en composé actif de formule (I) comprise entre 1,5 et 38 % en poids et de préférence entre 1,9 et 10 % en poids par rapport au poids total de la composition.

Les compositions selon l'invention peuvent se présenter sous diverses formes, notamment de solutions aqueuses ou hydroalcooliques, d'émulsions du type huile-dans-l'eau ou eau-dans-l'huile ou encore sous forme de gels émulsionnés.

De préférence, les compositions selon l'invention se présentent sous forme d'une lotion, d'une crème, d'un lait, d'un gel, d'un masque, de microsphères ou nanosphères ou de dispersions vésiculaires, ces vésicules pouvant être constituées de lipides ioniques (liposomes) et/ou de lipides non-ioniques.

Dans les émulsions, la phase grasse peut être constituée d'une huile végétale ou animale, d'une huile minérale ou encore d'une huile synthétique.

Parmi les huiles végétales ou animales modifiées ou non, on peut citer notamment l'huile d'amande douce, l'huile d'avocat, l'huile de ricin, l'huile d'olive, l'huile de jojoba, le perhydrosqualène, l'huile de calophyllum, la lanoline et ses dérivés, l'huile de tournesol, l'huile de germe de blé, l'huile de sésame, l'huile d'arachide, l'huile de pépins de raisin, l'huile de soja, l'huile de colza, l'huile de carthame, l'huile de coco, l'huile de maïs, l'huile de noisette, le beurre de karité, la graisse de shorea robusta, l'huile de palme et l'huile de noyau d'abricot.

Parmi les huiles minérales, on peut citer par exemple l'huile de vaseline et parmi les huiles synthétiques, les palmitates d'éthyle et d'isopropyle, les myristates d'alkyle tels que le myristate d'isopropyle, de butyle, de cétyle, le stéarate d'hexyle, les triglycérides des acides octanoïque et décanoïque (par exemple le produit vendu sous la dénomination de "MIGLYOL" par la Société DYNAMIT NOBEL), le ricinoléate de cétyle, l'octanoate de stéaryle (huile de purcellin) et le polyisobutène hydrogéné ainsi que des cires telles que l'ozokérite.

L'excipient gras peut également contenir certains composés considérés comme des produits gras à savoir des alcools à longues chaînes tels que l'alcool cétylique, l'alcool stéarylique, l'alcool myristique, l'alcool hydroxystéarylique, l'alcool oléique ou l'alcool isostéarylique.

Comme huile synthétique, on peut notamment citer les huiles de silicone. Parmi celles-ci, on utilise de préférence le cyclopentadiméthylsiloxane et notamment le produit vendu sous la dénomination de "VOLATIL SILICONE 7158" par la Société UNION CARBIDE, ainsi que l'alkyldiméthicone copolyol, en particulier le produit vendu sous la dénomination de "ABIL WE 09" par la Société GOLDSCHMIDT.

Les compositions cosmétiques ou dermatologiques selon l'invention peuvent bien entendu contenir d'autres ingrédients conventionnels tels que des agents humectants, des conservateurs, des colorants, des parfums, des agents de pénétration tels que le monoéthyléther de diéthylène glycol.

Ces compositions sont appliquées par voie topique en quantité correspondant aux doses d'application usuelles pour le type composition considérée (gels, crèmes, lotions, ...). Par exemple dans le cas d'une crème, on utilise de 0,5 à 3 mg et notamment de 1 à 2 mg de crème par $cm^2$ de peau et par application à raison d'une ou deux applications par jour.

Préparation des composés (3) et (4) par extraction alcoolique de Garnieria Spathulaefolia

Garnieria spathulaefolia est une plante appartenant à la famille des Protéacées et poussant en altitude (1000 m) dans les massifs péridotitiques du Nord de la Nouvelle-Calédonie.

L'extrait est fabriqué par macération, à trois reprises dans l'alcool à 96 °C, de la poudre grossière de feuilles, obtenue par broyage des feuilles de la plante séchée 15 heures à 55 °C.

Les phases alcooliques réunies sont alors évaporées à sec à partir de 200 g de poudre de feuilles, on obtient 32,1 g d'extrait (A), soit un rendement de 16 % (voir tableau I).

L'extrait (A) est additionné d'eau et soumis à une agitation pendant 2 heures, puis l'on filtre la suspension. La fraction soluble dans l'eau est extraite à l'acétate d'éthyle à 7 reprises. On prélève la fraction soluble dans l'acétate d'éthyle que l'on lave à l'eau puis évapore à sec pour obtenir 4 g d'un produit (B). Cette fraction (B) est soumise en partie (3 g) à une chromatographie sur colonne de silice en utilisant comme éluant un mélange AcOEt-$CH_2Cl_2$-MeOH 8:3:1 puis 0:12:1 puis 0:4:1. Cette chromatographie donne plusieurs fractions dont la fraction 77E qui est à son tour soumise à séparation par chromatographie sur silice en phase inverse. On obtient ainsi le composé (3) (66 mg) et le composé (4) (48

mg).

Ces deux composés ont les caractéristiques physico-chimiques suivantes :

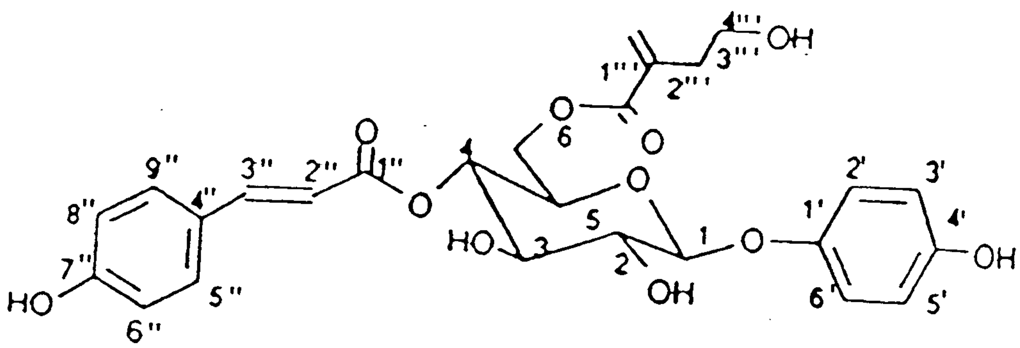

Composé (3) :

E,4-(p-hydroxy-cinnamoyl)-6-(4'''-hydroxy-2'''-méthylène butanoyl)-arbutoside

Formule brute $C_{26}H_{28}O_{11}$

$$[\alpha]_D^{25°C} = -14,6° \text{ (C=0,5 dans MeOH)}$$

P.m 516

Spectre UV

λ (EtOH) nm 211, 250, 313

Spectre infrarouge (KBr, $cm^{-1}$):1061, 1159, 1215, 1441, 1518, 1602, 1714, 2950.

Spectre de masse

(FAB):539 $(M+Na)^+$

Spectre RMN du $^1H$ (400 MHz, $CD_3OD$)

| δ (ppm) | | | J(Hz) | proton |
|---|---|---|---|---|
| 7,70 | 1H | d | 16 | H-3'' |
| 7,50 | 2H | d | 9,0 | H-5'',9'' |
| 6,95 | 2H | d | 9,0 | H-2',6' |
| 6,80 | 2H | d | 9,0 | H-6'',8'' |
| 6,70 | 2H | d | 9,0 | H-3',5' |
| 6,40 | 1H | d | 16 | H-2'' |
| 6,25 | 1H | d | 1,5 | = CHaHb |
| 5,75 | 1H | d | 1,5 | = CHaHb |
| 5,00 | 1H | t | 9,0 | H-4 |
| 4,85 | 1H | d | 8,0 | H-1(β) |
| 4,35 | 1H | dd | 13,3 | H-6a |
| 4,25 | 1H | dd | 13,6 | H-6b |
| 3,90 | 1H | m | | H-5 |
| 3,75 | 1H | t | 9,0 | H-3 |
| 3,65 | 2H | t | 6,5 | $CH_2$-$CH_2$OH |
| 3,55 | 1H | m | | H-2 |
| 2,55 | 2H | t | 6,5 | $CH_2$-$CH_2$OH |

Spectre RMN du $^{13}C$ (50 MHz, $CD_3OD$ δ ppm)

168,33(C-1''');167,98(C-1'');161,37(C-7'');154,00(C-1');152,12(C-4'); 147,42(C-3'');138,42(C-2''');131,31(C-5'',9'');128,17(= $CH_2$);127,10(C-4'');119,61(C-2',6');116,86(C-3',5');116,68(C-6'',8'');112,30(C-2);103,52(C-1)-

;75,58(C-4) ;75,03(C-3);73,30(C-2);72,33(C-5);64,40(C-6);61,57(C-4''');36,21(C-3''').

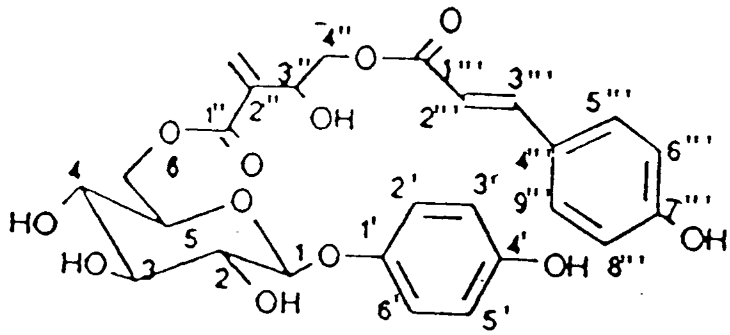

Composé (4) :

E,4''-(p-hydroxy-cinnamoyloxy)-6-(3'',4''-dihydroxy-2''-méthylène-butanoyl) arbutoside

Formule brute $C_{26}H_{28}O_{12}$

$$[\alpha]_D^{25°C} = -38,2° \text{ (C=0,5 dans MeOH)}$$

Spectre UV
λ max (EtOH) nm : 209, 250, 313
Spectre infrarouge (KBr, $cm^{-1}$)1082, 1180, 1216, 1271, 1518, 1595, 1715, 2950.
Spectre de masse
(FAB):555$(M+Na)^+$
Spectre RMN du $^1H$ (400MHz, $CD_3OD$)

| δ (ppm) | | | J(Hz) | proton |
|---|---|---|---|---|
| 7,55 | 1H | d | 16 | H-3'' |
| 7,37 | 2H | d | 9,0 | H-5''',9''' |
| 6,90 | 2H | d | 9,0 | H-2',6' |
| 6,75 | 2H | d | 9,0 | H-6''' ,8''' |
| 6,62 | 2H | d | 9,0 | H-3',5' |
| 6,37 | 1H | s | | = CHaHb |
| 6,28 | 1H | d | 16 | = H-2''' |
| 6,05 | 1H | s | | = CHaHb |
| 4,77 | 1H | m | | H-3'' |
| 4,72 | 1H | d | 8,0 | H-1 |
| 4,55 | 1H | dd | 12,3 | H-6a |
| 4,20 | 3H | m | | H-6b,$CH_2$-4'' |
| 3,65 | 1H | m | | H-5 |
| 3,42 | 2H | m | | H-2,3 |
| 3,35 | 1H | m | | H-4 |

Spectre RMN du $^{13}C$(50MHz$CD_3OD$ δppm)
168,90(C-1'');167,10(C-1''');161,53(C-7''');154,01(C-1');151,49(C-4');146,72(C-3''');141,62(C-2'');131,69(C-5''',9''');127,27(=$CH_2$);127,27(C-4''');119,62(C-2'-6');116,70(C-6''',8''');116,67(C-3',5');103,42(C-1);77,86(C-3)-;75,22(C-5);74,8-2(C-2);71,82(C-4);69,29(C-3'');68,12(C-4'');65,20(C-6).

TABLEAU I

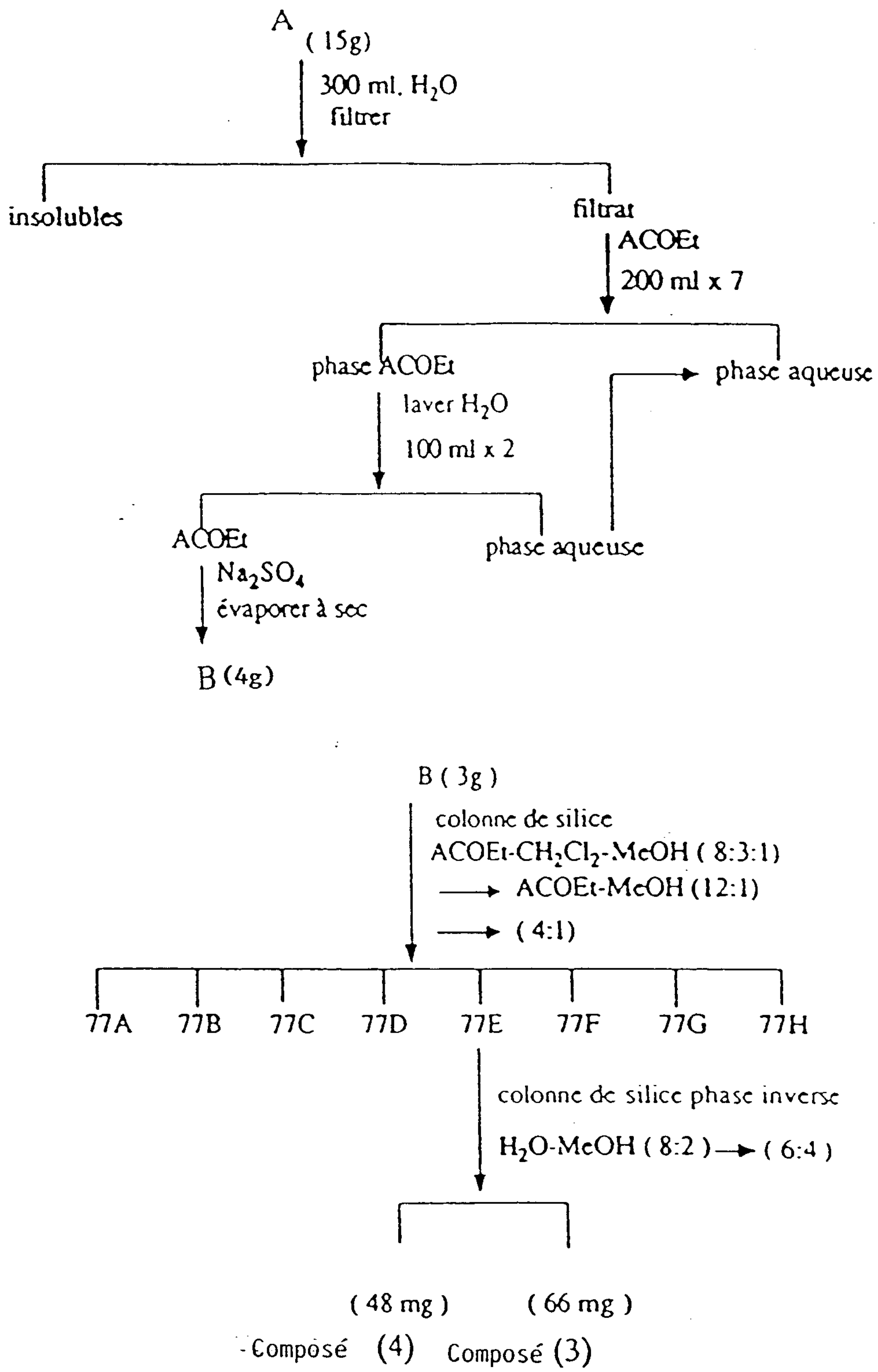
A ( 15g)
300 ml. $H_2O$
filtrer
insolubles
filtrat
ACOEt
200 ml x 7
phase ACOEt
laver $H_2O$
100 ml x 2
phase aqueuse
ACOEt
$Na_2SO_4$
évaporer à sec
phase aqueuse
B (4g)
B ( 3g )
colonne de silice
ACOEt-$CH_2Cl_2$-MeOH ( 8:3:1)
ACOEt-MeOH (12:1)
( 4:1)
77A
77B
77C
77D
77E
77F
77G
77H
colonne de silice phase inverse
$H_2O$-MeOH ( 8:2 ) ( 6:4 )
( 48 mg )
( 66 mg )
Composé (4)
Composé (3)

COMPOSITIONS COSMETIQUES OU DERMATOLOGIQUES

EXEMPLE 1 : Lotion

| | |
|---|---|
| - Alcool | 50 g |
| - Polyoxyéthylène glycol (PEG-8) | 30 g |
| - Ethoxy diglycol | 5 g |
| - Glycérine | 5 g |
| - Eau | 3 g |
| - 6-p-coumaroylarbutoside | 7 g |

EXEMPLE 2 : Emulsion eau-dans-l'huile

| | |
|---|---|
| - Glycérine | 10 g |
| - Propylène glycol | 5 g |
| - 6-p-coumaroylarbutoside | 3 g |
| - Cyclopentadimethylsiloxane | 20 g |
| - Polydiméthylsiloxane polyoxyéthylène | 3 g |
| - Parfum | 0,1 g |
| - Conservateurs | qs |
| - Eau | qsp. 100 g |

EXEMPLE 3 : Emulsion huile-dans-l'eau

| | |
|---|---|
| - Polyéthylèneglycoléther de l'alcool cétéarylique | 1 g |
| - Glycéryl stéarate | 3 g |
| - Coco caprylate/caprate (esters d'acides en $C_8$-$C_{10}$ et d'alcools gras en $C_{12}$-$C_{18}$) | 5 g |
| - Polymère acrylique (CARBOMER 934 vendu par la Société GOODRICH) | 0,3 g |
| - Triéthanolamine (50 %) | 0,9 g |
| - Alcool | 20 g |
| - 6-p-coumaroylarbutoside | 2 g |
| - Glycérine | 3 g |
| - Conservateur | qs |
| - Parfum | qs |
| - Eau | qsp. 100 g |

EXEMPLE 4 : Préparation de liposomes en formulation sérum

Dans un ballon rond de 1 litre, on pèse les produits suivants :

| | |
|---|---|
| - Lécithine de soja : lipoïd S75 (vendue par la Société Lipoïd) | 3,0 g |
| - 6-p-coumaroylarbutoside | 0,3 g |

que l'on dissout dans 100 ml d'un mélange de solvants chloroforme - méthanol dans le rapport 2/1.

On évapore le solvant à l'aide d'un évaporateur rotatif et on élimine les dernières traces de solvant par passage à la pompe à palettes pendant 1 heure. On met en contact l'association des lipides obtenue avec 60 g d'eau déminéralisée contenant 1 g de glycérine, et on homogénéise le mélange à 40 °C, à l'aide d'un homogénéisateur Virtis.

On termine la formule en ajoutant les produits suivants :

| | |
|---|---|
| - Parahydroxybenzoate de méthyle | 0,2 g |
| - Mélange d'acides carboxyvinyliques "Carbopol 940" | 0,1 g |
| - Triéthanolamine | q.s. pH = 6 |
| - Eau | qsp. 100 g |

On obtient ainsi un sérum opalescent fluide mais ne s'écoulant pas.

EXEMPLE 5 : Préparation de vésicules non ioniques en formulation crème

Dans un ballon rond de 100 ml, on pèse les produits suivants :

- Lipide non-ionique de formule :

$$C_{12}H_{25}-[OC_2H_3(R)]-O-[C_3H_5(OH)-O]_{\bar{n}}-H$$

où $-OC_2H_3(R)-$ est constitué par un mélange des radicaux :

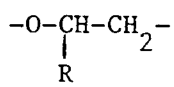 et 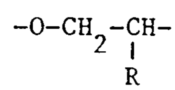 ;

où $-C_3H_5(OH)-O-$ est constitué par un mélange des radicaux :

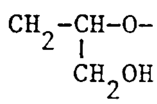 et 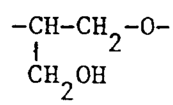 ;

où $\bar{n}$ = 6 ;

et où R est un mélange des radicaux $C_{14}H_{29}$

| | |
|---|---|
| et $C_{16}H_{33}$ | 0,9 g |
| - Acylglutamate de sodium HS21 (vendu par la Société Ajinomoto) | 0,1 g |
| - 6-p-coumaroylarbutoside | 0,1 g |

que l'on dissout dans 30 ml d'un mélange de solvants chloroforme-méthanol dans le rapport 2/1.

On évapore le solvant à l'aide d'un évaporateur rotatif et on élimine les dernières traces de solvant par passage à la pompe à palettes pendant 1 heure.

On met au contact l'association de lipides obtenue avec 40 g d'eau déminéralisée contenant 3 g de glycérine, et on homogénéise le mélange à 40 °C, à l'aide d'un homogénéisateur Virtis.

On ajoute ensuite 10 g de perhydrosqualène et on homogénéise le mélange à la température ambiante à l'aide du Virtis.

On finit la formule en ajoutant les produits suivants :

| | |
|---|---|
| - Parahydroxybenzoate de méthyle | 0,2 g |
| - Mélange d'acides carboxyvinyliques "Carbopol 940" | 0,4 g |
| - Triéthanolamine | q.s. pH = 6 |
| - Eau | qsp. 100 g |

On obtient ainsi une crème blanche, épaisse.

## Revendications

**1.** Composition cosmétique ou dermatologique à action dépigmentante, caractérisée par le fait qu'elle contient en tant que principe actif, au moins un dérivé d'arbutoside correspondant à la formule générale suivante :

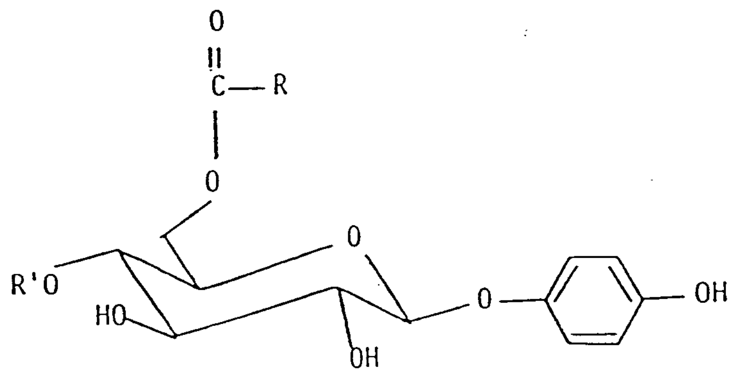

(I)

dans laquelle :
R représente un reste insaturé hydroxylé aliphatique ou aromatique ayant de 4 à 16 atomes de carbone, et
R' représente un atome d'hydrogène ou le radical

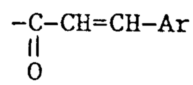

Ar représentant un radical phényle éventuellement substitué par un ou plusieurs groupes hydroxyles.

**2.** Composition selon la revendication 1, caractérisée par le fait que le reste insaturé hydroxylé aliphatique ou aromatique ayant de 4 à 16 atomes de carbone est choisi parmi les radicaux :

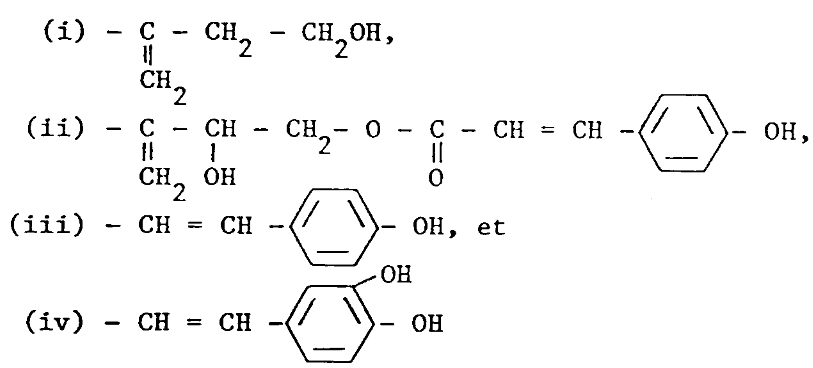

(i), (ii), (iii), et (iv)

**3.** Composition selon la revendication 1 ou 2, caractérisée par le fait que le dérivé d'arbutoside est choisi parmi :
6-p-coumaroylarbutoside,
6-caffeoylarbutoside,
E,4-(p-hydroxy-cinnamoyl)-6-(4'''-hydroxy-2'''méthylène butanoyl)-arbutoside, et
E,4''-(p-hydroxy-cinnamoyloxy)-6-(3'',4''-dihydroxy-2''méthylène butanoyl)-arbutoside.

**4.** Composition selon l'une quelconque des revendications précédentes, caractérisée par le fait que le dérivé d'arbutoside est présent à une concentration comprise entre 1,5 et 38 % en poids par rapport au

poids total de la composition.

5. Composition selon l'une quelconque des revendications précédentes, caractérisée par le fait que le dérivé d'arbutoside est présent à une concentration comprise entre 1,9 et 10 % en poids par rapport au poids total de la composition.

6. Composition selon l'une quelconque des revendications précédentes, caractérisée par le fait qu'elle se présente sous forme d'une lotion, d'une crème, d'un lait, d'un gel, d'un masque, de microsphères ou nanosphères ou de dispersions vésiculaires constituées de lipides ioniques (liposomes) et/ou de lipides non ioniques.

7. Composition selon l'une quelconque des revendications précédentes, caractérisée par le fait qu'elle contient en outre au moins un ingrédient cosmétique ou dermatologique choisi parmi les agents humectants, les agents conservateurs, les colorants, les parfums et les agents de pénétration.

## Claims

1. Cosmetic or dermatological composition having a depigmenting action, characterized in that it contains as active principle at least one arbutoside derivative corresponding to the following general formula:

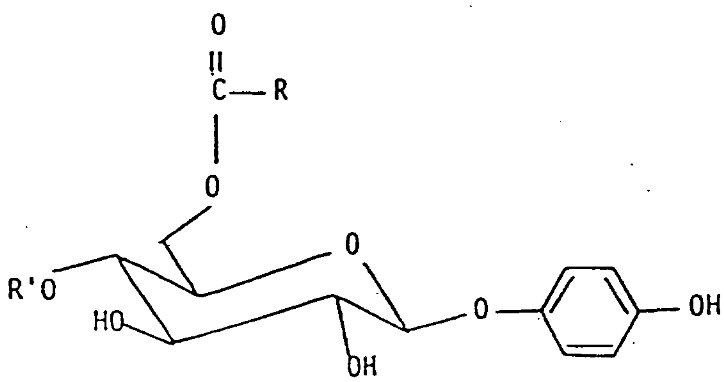

(I)

in which:
R represents an unsaturated hydroxylated aliphatic or aromatic residue having from 4 to 16 carbon atoms, and
R' represents a hydrogen atom or a radical

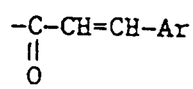

Ar representing a phenyl radical optionally substituted with one or more hydroxyl groups.

2. Composition according to Claim 1, characterized in that the unsaturated hydroxylated aliphatic or aromatic residue having from 4 to 16 carbon atoms is chosen from the radicals:

(i)
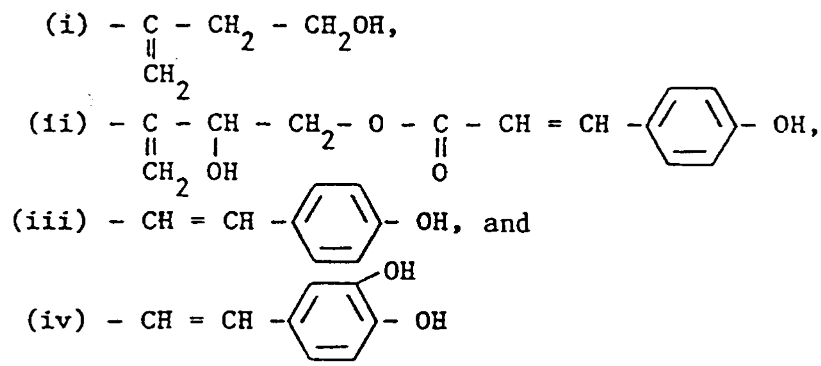
(ii)
(iii) and
(iv)

3. Composition according to Claim 1 or 2, characterized in that the arbutoside derivative is chosen from:
6-p-coumaroylarbutoside,
6-caffeoylarbutoside,
(E)-4-(p-hydroxycinnamoyl)-6-(4'''-hydroxy-2'''-methylenebutanoyl)arbutoside, and
(E)-4''-(p-hydroxycinnamoyloxy)-6-(3'',4''-dihydroxy-2''-methylenebutanoyl)arbutoside.

4. Composition according to any one of the preceding claims, characterized in that the arbutoside derivative is present at a concentration of between 1.5 and 38% by weight relative to the total weight of the composition.

5. Composition according to any one of the preceding claims, characterized in that the arbutoside derivative is present at a concentration of between 1.9 and 10% by weight relative to the total weight of the composition.

6. Composition according to any one of the preceding claims, characterized in that it takes the form of a lotion, cream, milk, gel, mask, microspheres or nanospheres or vesicular dispersions consisting of ionic lipids (liposomes) and/or nonionic lipids.

7. Composition according to any one of the preceding claims, characterized in that it contains, in addition, at least one cosmetic or dermatological ingredient chosen from humectants, preservatives, colorants, perfumes and penetrating agents.

**Patentansprüche**

1. Kosmetische oder dermatologische Zubereitung mit depigmentierender Wirkung, welche dadurch gekennzeichnet ist, daß sie als Wirkstoff mindestens ein Arbutosidderivat enthält, welches der folgenden allgemeinen Formel entspricht:

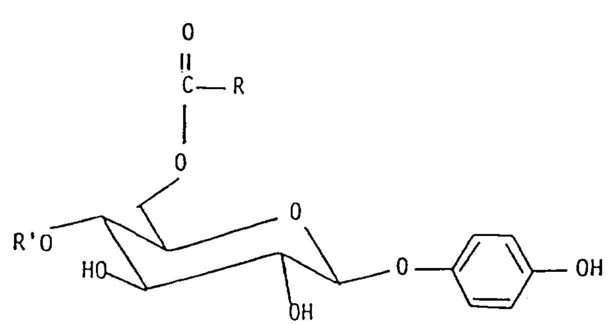
(I),

in der

R einen ungesättigten, hydroxylierten, aliphatischen oder aromatischen Rest mit 4 bis 16 Kohlenstoffatomen darstellt und

R' ein Wasserstoffatom oder den Rest -C( = O)-CH = CH-Ar bedeutet, wobei Ar einen gegebenenfalls mit einer oder mehreren Hydroxylgruppen substituierten Phenylrest darstellt.

**2.** Zubereitung gemäß Anspruch 1, dadurch gekennzeichnet, daß der ungesättigte, hydroxylierte, aliphatische oder aromatische Rest mit 4 bis 16 Kohlenstoffatomen unter den folgenden Resten ausgewählt ist:

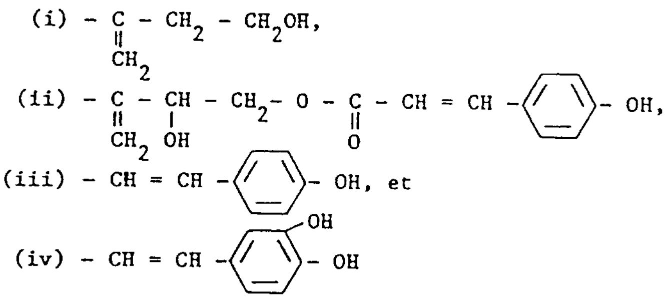

(i)

(ii)

(iii) et

(iv)

**3.** Zubereitung gemäß Anspruch 1, dadurch gekennzeichnet, daß das Arbutosidderivat aus den folgenden ausgewählt ist:
6-p-Kumaroylarbutosid,
6-Kaffeesäure-Arbutosid,
E,4-(p-Hydroyxcinnamoyl)-6-(4'''-hydroxy-2'''-methylenbutanoyl)-arbutosid und
E,4''-(p-Hydroxycinnamoyloxy)-6-(3'',4''-dihydroxy-2''-methylenbutanoyl)-arbutosid.

**4.** Zubereitung gemäß einem der vorstehenden Ansprüche, dadurch gekennzeichnet, daß das Arbutosidderivat in einer Konzentration von 1,5 bis 38 Gew.-%, bezogen auf das Gesamtgewicht der Zubereitung, vorliegt.

**5.** Zubereitung gemäß einem der vorstehenden Ansprüche, dadurch gekennzeichnet, daß das Arbutosidderivat in einer Konzentration von 1,9 bis 10 Gew.- %, bezogen auf das Gesamtgewicht der Zubereitung, vorliegt.

**6.** Zubereitung gemäß einem der vorstehenden Ansprüche, dadurch gekennzeichnet, daß sie in Form einer Lotion, einer Creme, einer Milch, eines Gels, einer Maske oder in Form von Mikro- oder Nanokügelchen oder einer Vesikeldispersion vorliegt, wobei die Vesikel aus ionischen Lipiden (Liposomen) und/oder nichtionischen Lipiden bestehen.

**7.** Zubereitung gemäß einem der vorstehenden Ansprüche, dadurch gekennzeichnet, daß sie zusätzlich mindestens einen weiteren kosmetischen oder dermatologischen Hilfsstoff enthält, der aus Feuchthaltemitteln, Konservierungsmitteln, Farbstoffen, Parfümen und Penetrationsverbesserern ausgewählt ist.